# EUROPEAN PATENT SPECIFICATION

(11) **EP 4 751 676 B1**
(45) Date of publication and mention of the grant of the patent: **09.12.2026**
(21) Application number: 25219696.9
(22) Date of filing: 01.12.2025
(51) Int. Cl.: A61B 34/10, A61B 18/14, A61B 34/20

(54) **COMPOUND ABLATION INDICATION OVER ANATOMICAL MAP**
VERBINDUNGSABLATIONSANZEIGE ÜBER EINE ANATOMISCHE KARTE
INDICATION D'ABLATION DE COMPOSÉ SUR UNE CARTE ANATOMIQUE

(30) Priority: 02.12.2024 US 202463726831 P; 05.11.2025 US 202519380362
(43) Date of publication of application: 03.06.2026
(73) Proprietor: Biosense Webster (Israel) Ltd., 2066717 Yokneam (IL)
(72) Inventor: GOLDBERG, Stanislav, 2066717 Yokneam (IL); COHEN, Yaniv, 2066717 Yokneam (IL); COHN, Goren, 2066717 Yokneam (IL); KLEMM, Ofer, 2066717 Yokneam (IL); BREM, Erez Henri, 2066717 Yokneam (IL)
(74) Representative: Carpmaels & Ransford LLP

(56) References cited:
- EP-A1- 2 952 151
- EP-A1- 3 797 691
- CA-A1- 2 946 690

## Description

### FIELD OF THE DISCLOSURE

This disclosure relates generally to cardiac ablation, and specifically to a system and method for real-time planning and monitoring of cardiac ablation using an anatomical map.

### BACKGROUND OF THE DISCLOSURE

Providing indications of ablation points on an anatomical map of an inner wall of a cardiac chamber was previously proposed in the patent literature. For example, U.S. Patent 10,588,692 describes how a gap between a plurality of ablation sites in a heart is found by projecting the locations of the sites in a three-dimensional coordinate system onto a simulation plane, identifying a set of shortest three-dimensional paths that correspond to two-dimensional connections between pairs of the projected locations of the sites, and reporting a gap as a longest one of the set.

CA2946690 A1 discloses that a gap between a plurality of ablation sites in a heart that hinders electrical propagation therethrough is found by projecting the locations of the sites in a 3-dimensional coordinate system onto a simulation plane, identifying a set of 5 shortest 3-dimensional paths that correspond to 2-dimensional connections between pairs of the projected locations of the sites, and reporting a gap as a longest one of the set.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Fig. 2 is a schematic pictorial illustration of ablation indications over a map surface of a cardiac chamber after the chamber's ablation by a loop catheter, in accordance with an example of the present disclosure;
Fig. 3 is an ablation map of a cardiac chamber derived using the disclosed compound ablation indication algorithm, in accordance with an example of the present disclosure; and
Fig. 4 is a flow chart that schematically illustrates a method for generating an ablation map of a cardiac chamber, in accordance with an example of the present disclosure.

### DETAILED DESCRIPTION OF EXAMPLES

### OVERVIEW

In a cardiac ablation procedure, such as pulmonary vein isolation (PVI) to treat atrial fibrillation, a physician ablates tissue in a specific anatomical region (e.g., over an entire circumference of an ostium of a PV). The ablation, such as one using a pulsed-field ablation (PFA) technique, may require several iterations to fully cover the entire circumference of the ostium. Each iteration requires moving a multi-electrode ablation catheter to areas deemed by the physician as insufficiently ablated or not ablated at all.

The physician may be aided with an ablation map comprising an anatomical 3D map that displays ablation tags graphically encoded to indicate where the ablation was already performed. The ablation tags are located on the anatomical 3D map surface, including ones initially located sufficiently near the anatomical 3D map surface and projected onto the surface.

However, the projection of ablation data onto the anatomical map surface can lead to an inaccurate representation of the locations and continuity of the ablation indicators on the anatomy's surface due to various reasons:
1. The anatomical map surface may not always represent the actual anatomy due to different factors (e.g., due to catheter movement as a result of respiration, low number of anatomic data points collected during mapping, errors in mapping, etc.)
2. The ablation catheter may move (e.g., push) the local anatomical structure from its normal position by applying force on the tissue prior or during ablation.
3. Complex multi-electrode catheters (e.g., loop or multi-spine such as flower or basket catheters), especially catheters that perform bipolar ablation, may produce ablation tags that are not projected properly by nearest-neighbor projection primarily due to the following reasons:
   3.1. Bipolar ablations produce a flow of electrical current (or electromagnetic field in PFA) between the ablation electrodes, contrary, for example, to unipolar focal ablation that produce a flow of electrical current (or electromagnetic field in PFA) between ablation electrodes and the nearest tissue and therefore more suitable for nearest-neighbor projection.
   3.2. Misrepresentation over projected indicators (e.g., graphically encoded grid tags) on the map of the ablation gap between certain electrodes that were not a part of a bipolar ablation sequence, such as the proximal and distal electrodes of the loop catheter.
   3.3. Lack of continuity in projected ablation indicators (e.g., grid tags) between that represent a pair of electrodes those that were a part of bipolar ablation sequence. Such lack of continuity may give the physician an impression of ablation gaps in this area, mainly because of the anatomy surface mapping artefacts as mentioned in paragraphs 3.1 and 3.2 above.

Another issue contributing to inaccurate representation of locations and ablation continuity in a map (such as wrongly presenting ablation gaps) is the small number of ablation data points, which is due to the clinical motivation to achieve continuous blocking of arrhythmia while minimizing damage to cardiac tissue. As a result, each erroneous ablation location on the map can be significant.

The abovementioned issues, and others, can cause the ablation indicators (e.g., graphically encoded tags) to either fail to indicate an existing ablation gap or falsely indicate an ablation gap where it does not exist. Such errors make it hard for a physician to decide where further ablation is indicated, or ablation is not required.

Examples of the present disclosure described herein provide ablation mapping techniques that both consistently show ablation gaps and largely eliminate the inaccuracies caused by the issues listed above.

The present invention is defined in the appended claims.

In one example, a processor runs the disclosed algorithm steps:
1. Projecting electrode locations of multi-electrode catheters onto the anatomical map surface, using any suitable projection method, only if the distance to the map surface is smaller than a threshold projection distance (e.g., smaller than 7 mm).
2. Connecting the projected electrode locations on the anatomical map surface using a shortest path algorithm (such as Dijkstra geodesic).
3. Creating a visual indication of the connected path between pairs of projected electrode locations by limiting the geodesic path between the projected electrodes' locations within clinically acceptable range (e.g., under a predefined threshold path length). This step gives more certainty to nearest electrodes having created a continuous ablation than may otherwise be indicated by the map location.
4. Creating a visual indication of a potential gap along the path if the connection between the projected locations of electrodes does not result in effective ablation due to catheter geometry (for electrodes that weren't a part of bipolar ablation sequence and do not result in effective ablation between them). When such a step is applied to a loop ablation catheter, the processor indicates a path between projected proximal-distal electrode locations as an ablation gap for proximal-distal electrodes that were a part of ablation sequence. For example, a physician could turn on only 2-8 electrodes instead of 1-10, so the gap would be between electrodes 2 and 8.
5. Repeating steps 1-4 for each ablation instance indicates a connected path and overrides prior indications of a potential gap along the same path. As more ablation instances are analyzed, this step removes probable false positive indications of ablation gaps from the map.

While the disclosed technique is presented for a loop catheter, the disclosed technique is applicable, *mutatis mutandis,* to other catheter geometries, such as multi-spline, flower, grid or basket.

The disclosed technique may also be applied, *mutatis mutandis,* to improve diagnostic mapping accuracy or reduce diagnostic mapping time, since mapping procedures usually acquire far more data points, and use more flexible catheters, therefore resulting in mapping tag location errors still existing in these diagnostic maps.

### SYSTEM DESCRIPTION

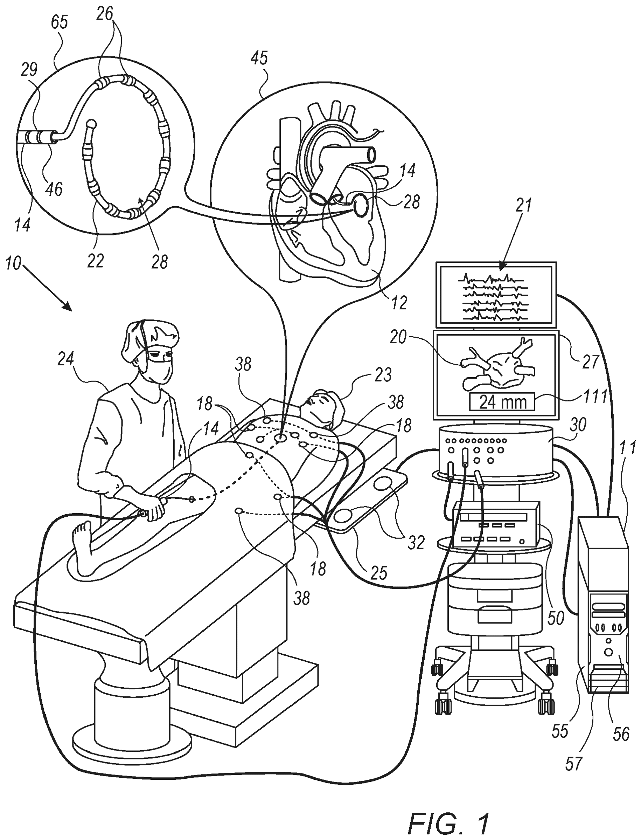

Fig. 1 is a schematic, pictorial illustration of a catheter-based electro-anatomical (EA) mapping and ablation system 10, in accordance with an example of the present disclosure.

System 10 includes a loop ablation catheter 14, which is percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Afterward, catheter 14 can be inserted into the delivery sheath catheter to arrive at the desired location. The plurality of catheters may include a catheter dedicated to pacing, a catheter for sensing intracardiac electrogram signals, a catheter dedicated to ablating and/or a catheter dedicated to both EA mapping and ablating. Loop catheter 14, illustrated herein, is configured for sensing bipolar electrograms and applying PFA. Physician 24 brings a distal end assembly 28 of catheter 14 into contact with the heart wall to ablate a target site in heart 12.

As seen in inset 65, distal end assembly 28 includes multiple electrodes 26 distributed over a curved spline 22. Catheter 14 may include a position sensor 29, embedded in or near distal end assembly 28 on a shaft 46 of catheter 14, to track the position and orientation of distal end assembly 28. Optionally, and preferably, position sensor 29 is a magnetic-based position sensor with three magnetic coils for sensing three-dimensional (3D) position and orientation.

The magnetic-based position sensor 29 may be operated with a location pad 25 that includes a plurality of magnetic coils 32 configured to generate magnetic fields in a predefined working volume. The real-time position of distal end assembly 28 of catheter 14 may be tracked based on magnetic fields generated with location pad 25 and sensed by magnetic-based position sensor 29. Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm and/or may be electrically connected to a standalone pacer.

System 10 includes an ablation energy generator 50 adapted to conduct ablative energy to one or more electrodes 26 configured for ablation. The energy produced by ablation energy generator 50 may include, but is not limited to, radiofrequency (RF) energy or pulse field (PF) energy, including monopolar or bipolar high-voltage DC pulses (i.e., PFA).

The patient interface unit (PIU) 30 is an interface configured to establish electrical communication between catheters, electrophysiological equipment, power supply, and a workstation 55 to control system 10 operation and receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO^{™} 3 System, available from Biosense Webster, Inc., 31A Technology Drive, Irvine, CA 92618.

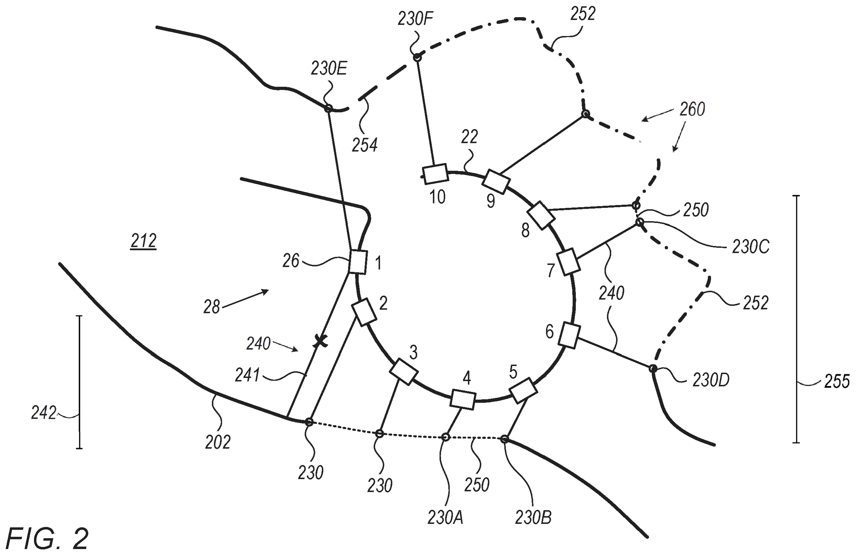

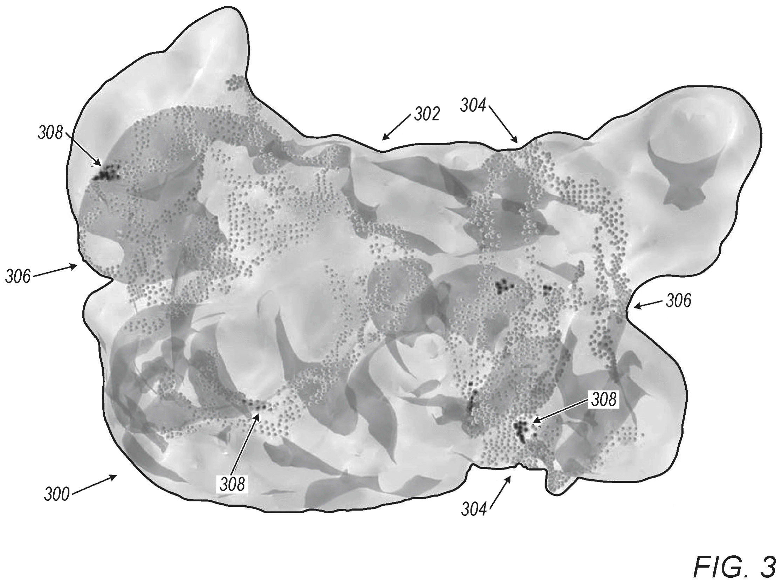

In the disclosed example, processor 56 runs an algorithm that generates an ablation map showing ablation gaps with increased confidence, as shown in Figs. 2 and 3.

In some examples, processor 56 typically comprises a general-purpose computer programmed in software to perform the functions described herein. The software may be downloaded to the computer in electronic form over a network, for example, or it may, alternatively or additionally, be provided and/or stored on non-transitory tangible media, such as magnetic, optical, or electronic memory.

This configuration of system 10 is shown by way of example to illustrate certain problems addressed by examples of the present disclosure and to demonstrate the application of these examples in enhancing the performance of such a system. However, examples of the present disclosure are not limited to this specific example system, and the principles described herein may similarly be applied to other medical systems. For example, other multi-electrode catheter types may be used, such as a basket catheter.

### COMPOUND INDICATION OF ABLATION

As noted above, the disclosed technique provides an algorithm to generate an ablation map showing ablation gaps with an increased confidence level, as has been determined by retrospective studies. Some of the algorithm steps are described in Fig. 2, an analysis of one ablation instance, which shows a schematic pictorial illustration of ablation indications (250, 252, 254) over a map surface 202 of a cardiac chamber after the chamber's ablation by a loop catheter 28, in accordance with an example of the present disclosure.

As seen, the locations of the ablating electrodes 26 are projected (240) onto locations 230 on map surface 202. Electrode 1 projection 240 is not considered, since its calculated projection distance (241) is above the threshold projection distance 242 (e.g., larger than 7 mm).

An edge electrode such as proximal electrode 1 may have two projections, one relative to that of electrode 2 and another relative to the projection of electrode 10. Both projections are considered in this disclosure.

The disclosed algorithm divides ablation indications on map surface 202 into three groups:
Group 1: indications 250 between adjacent electrode projected locations (e.g., 230A and 230B) on map surface 202, indicating paths 260 of continuous ablation.
Group 2: indications 252 between adjacent electrode projected locations (e.g., 230C and 230D) on map surface 202, also indicating paths 260 of continuous ablation, despite the large length of the path. To this end, a predefined path length threshold 255 is set high enough. This crucial step gives more certainty to the nearest electrodes, such as (6, 7), creating a continuous ablation that may be considered by the length between the projected locations, e.g., 230C and 230D.
   Indications 250 and 252 are collectively named "first indications.
Group 3: second indications 254 between adjacent electrode projected locations (e.g., 230E and 230F) on map surface 202, the indicating paths of ablation gaps due to ineffective PFA between certain adjacent electrodes. In the case of Fig. 2 of loop catheter 28, gap indication 254 is between the projected locations of proximal electrode 1 and distal electrode 10 of the catheter.

An ablation session includes many instances of the sort shown in Fig. 2. An ablation map based on the above analysis applied to multiple ablation instances is provided in Fig. 3.

### ABLATION MAP BASED ON COMPOUND ABLATION INDICATIONS

Fig. 3 is an ablation map 300 of a cardiac chamber derived using the disclosed compound ablation indication algorithm, in accordance with an example of the present disclosure. Ablation map 300 is defined herein as an anatomical map 302 superimposed/overlayed with grid ablation tags 304.

In the context of this disclosure, ablation tags 304, in their displayed grid, are associated with grid ablation points defined by the 3D mapping coordinate system. The ablation point grid divides the 3D space densely (e.g., by sub-millimeter). The density of the ablation tags is based on a dipole model of the PFA energy deposition around each electrode.

Map 300 is also based on indications 250, 252, and 254 of Fig. 2 to show regions of ablation tags 304 graphically encoded 306 to indicate continuous ablation and graphically encoded 308 to indicate ablation gaps.

Ablation map 300 shows ablation grid tags 308 that are graphically encoded to indicate possible ablation gaps. Indication 254 of Fig. 2 is an example of a derivation for graphical encoding 308.

As noted above, map 300 is also based on indications 252 of Fig. 2, which are used to minimize erroneous gap identifications. Map 300 assists the physician to fully complete the PFA treatment and minimize extra ablation.

Map 300 can be generated offline using stored data, or generated during a clinical ablation procedure and updated in real time as the ablation procedure advances.

### METHOD OF INDICATION OF ABLATION ON ANATOMICAL MAP

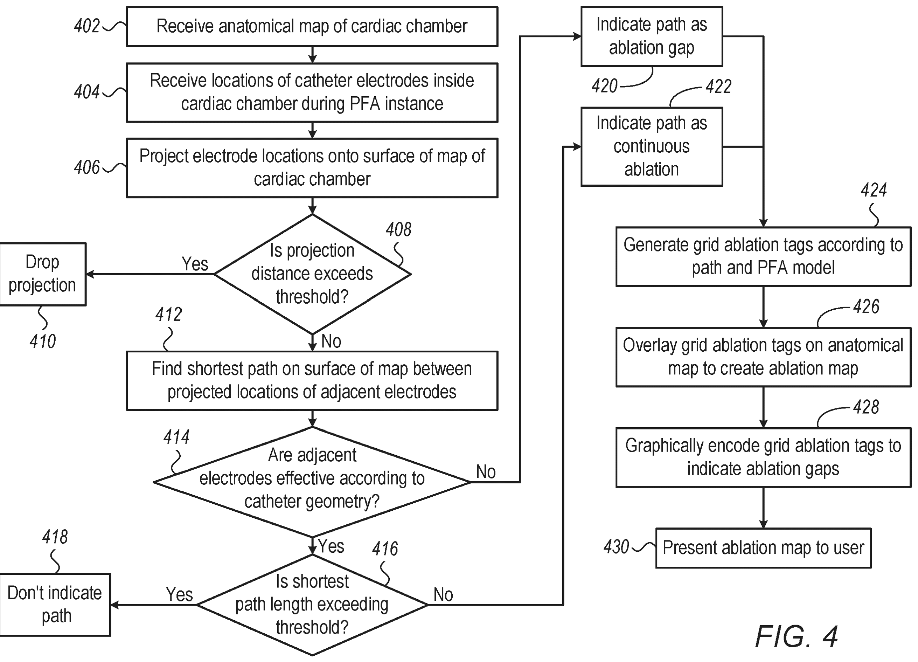

Fig. 4 is a flow chart that schematically illustrates a method for generating an ablation map of a cardiac chamber, in accordance with an example of the present disclosure. The algorithm, according to the presented example, carries out a process that begins with processor 56 receiving anatomical map 302 of wall tissue of a portion of a cardiac chamber, at a map receiving step 402.

The processor further receives the location of catheter electrodes 26 inside the cardiac chamber during an ablation instance, at a receiving electrode locations step 404.

Next, the processor projects electrode locations onto surface 202 of anatomical map 302, at a projection step 406.

At checking step 408, the processor checks the distance (241) of each projection (i.e., the distance between electrode location and projected location). If this distance exceeds a predefined distance threshold, the processor drops the projection from consideration, at projection dropping step 410. This step aims to increase confidence in the disclosed method, since accepting exceedingly long projections may cause the algorithm to overestimate ablation continuity, thereby overlooking ablation gaps.

For valid projections, i.e., where the distance (241) of the projection is under the predefined threshold projection distance 242, the processor finds the shortest paths on map 302 between the projected locations of adjacent electrodes, as seen in Fig. 2, in path finding step 412.

In step 414, the processor checks if, according to the catheter geometry, there are projected paths found in step 412 that do not represent effective ablation. For the example of loop catheter 28, a path that connects between projected proximal (1) and distal (10) electrode locations 230E and 230F indicates (254) an ablation gap. A gap between proximal and distal electrodes is intrinsic to many catheter types, thus, regardless of the projections properties, there is no ablation between these electrodes.

In indicating step 420, the processor indicates an ineffective path (254) as a possible ablation gap.

After considering any ineffective electrode pairs in step 414, the processor checks if the shortest paths between any other projected locations of adjacent electrodes exceed a predefined threshold path length (255).

If the answer is "yes," the processor avoids indicating that path, at step 418. This step aims to increase confidence in the disclosed model, since indicating excessively long paths may cause the algorithm to miss ablation gaps.

If the answer to step 414 is "no," the processor indicates the checked paths as continuous ablation paths, at path indication step 422.

At ablation tag generation step 424, the processor generates grid ablation tags 304 based on the indications of steps 420 and 422. The tags are distributed according to a model of PFA energy density in the tissue (e.g., a dipole model).

At overlaying step 426, the processor overlays (i.e., superimposes) grid ablation tags 304 on anatomical map 302 to generate ablation map 300.

The processor graphically encodes (306, 308) the grid ablation tags 304 to indicate ablation gaps, at graphical encoding step 428. Graphically encoding the tag ablation map may comprise coloring ablation tags over a continuous region with one color and coloring ablation tags over an ablation gap with another color.

Finally, the processor presents the graphically encoded ablation map 300 to a user on the display device 27, at ablation map presentation step 430.

The flow chart of Fig. 4 is simplified to describe a single instance of ablation while, in an actual procedure, many ablation instances may occur, leading the processor to update ablation map 300 multiple times during the procedure. As more ablation instances are considered, a region encoded (308) as having a gap may become one encoded (306) as continuous. A final ablation map 300 will show no gaps if the ablation session was fully executed.

Map 300 may be generated using offline data, or generated during a clinical ablation procedure and updated in real time as the ablation procedure advances.

It will be appreciated that the present disclosure is not limited to what has been shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art.

## Claims

1. A system (10), comprising:
a display device (27); and
a processor (56), which is configured to:
receive an anatomical map (302) of wall tissue of at least a portion of a cardiac chamber; and
**characterized in that** the processor is further configured to:
receive locations of electrodes (26) of a catheter (14) inside the cardiac chamber during the application of ablation;
project (240) at least some of the locations of the electrodes onto a surface (202) of the map;
find shortest paths between pairs of projected locations (230) of adjacent electrodes (26);
indicate some of the paths with first indications (250, 252) of continuous ablation, when the paths (260) have path lengths that are below a given threshold path length (255);
indicate others of the paths with second indications (254) of ablation gaps based on the catheter geometry;
generate grid ablation tags (304) according to the first and second indications and to an ablation model;
superimpose the grid ablation tags (304) over the anatomical map (302) to generate an ablation map (300);
graphically encode (306, 308) the ablation map according to the first and second indications of the paths; and
present the graphically encoded (306, 308) ablation map (300) to a user on the display device (27).

2. The system according to claim 1, wherein the processor is configured to project (240) the locations by checking a distance (241) between the location and the respective projected location (230), and discarding one or more of the projected (240) locations whose distance (241) exceeds a threshold projection distance (242).

3. The system according to any of claims 1 and 2, wherein the processor is configured to find the shortest paths (260) by finding geodesic curves on the surface (202) of the anatomical map.

4. The system according to any of claims 1 through 3, wherein the processor is configured to indicate (254) the paths with the second indications of ablation gaps based on the catheter (14) geometry by, for a loop (28) catheter, indicating a path generated by projection of a proximal-most electrode (26) of the catheter and a distal-most electrode (26) of the catheter as an ablation gap.

5. The system according to any of claims 1 through 4, wherein the processor is configured to generate the grid ablation tags (304) by using a dipole model.

6. The system according to any of claims 1 through 5, wherein the processor is configured to graphically encode (306, 308) the ablation map (300) by coloring ablation tags (304) over a continuous region with one color and coloring ablation tags over an ablation gap with another color.

7. The system according to any of claims 1 through 6, wherein the anatomical map (302) is an electroanatomical (EA) map.

## Patentansprüche

1. System (10), umfassend:
eine Anzeigevorrichtung (27); und
einen Prozessor (56), der konfiguriert ist zum:
Empfangen einer anatomischen Karte (302) von Wandgewebe von mindestens einem Abschnitt einer Herzkammer; und
**dadurch gekennzeichnet, dass** der Prozessor ferner konfiguriert ist zum:
Empfangen von Positionen von Elektroden (26) eines Katheters (14) innerhalb der Herzkammer während der Anwendung von Ablation;
Projizieren (240) von mindestens einigen der Positionen der Elektroden auf eine Oberfläche (202) der Karte;
Finden kürzester Pfade zwischen Paaren von projizierten Positionen (230) von angrenzenden Elektroden (26);
Angeben einiger der Pfade mit ersten Angaben (250, 252) von kontinuierlicher Ablation, wenn die Pfade (260) Pfadlängen aufweisen, die unter einer gegebenen Schwellenpfadlänge (255) liegen;
Angeben anderer der Pfade mit zweiten Angaben (254) von Ablationslücken basierend auf der Kathetergeometrie;
Erzeugen von Gitterablationsmarkierungen (304) gemäß den ersten und zweiten Angaben und einem Ablationsmodell;
Überlagern der Gitterablationsmarkierungen (304) über die anatomische Karte (302), um eine Ablationskarte (300) zu erzeugen;
grafisches Codieren (306, 308) der Ablationskarte gemäß den ersten und zweiten Angaben der Pfade; und
Darstellen der grafisch codierten (306, 308) Ablationskarte (300) für einen Benutzer auf der Anzeigevorrichtung (27).

2. System nach Anspruch 1, wobei der Prozessor konfiguriert ist, um die Positionen zu projizieren (240), durch Prüfen eines Abstands (241) zwischen der Position und der jeweiligen projizierten Position (230) und Verwerfen einer oder mehrerer der projizierten (240) Positionen, deren Abstand (241) einen Schwellenprojektionsabstand (242) überschreitet.

3. System nach einem der Ansprüche 1 und 2, wobei der Prozessor konfiguriert ist, um die kürzesten Pfade (260) zu finden, durch Finden geodätischer Kurven auf der Oberfläche (202) der anatomischen Karte.

4. System nach einem der Ansprüche 1 bis einschließlich 3, wobei der Prozessor konfiguriert ist, um die Pfade mit den zweiten Angaben von Ablationslücken basierend auf der Geometrie des Katheters (14) anzugeben (254), durch, für einen Schleifenkatheter (28), Angeben eines Pfads, der durch Projektion einer proximalsten Elektrode (26) des Katheters und einer distalsten Elektrode (26) des Katheters erzeugt wird, als eine Ablationslücke.

5. System nach einem der Ansprüche 1 bis einschließlich 4, wobei der Prozessor konfiguriert ist, um die Gitterablationsmarkierungen (304) durch Verwenden eines Dipolmodells zu erzeugen.

6. System nach einem der Ansprüche 1 bis einschließlich 5, wobei der Prozessor konfiguriert ist, um die Ablationskarte (300) durch Einfärben von Ablationsmarkierungen (304) über einen zusammenhängenden Bereich mit einer Farbe und Einfärben von Ablationsmarkierungen über eine Ablationslücke mit einer anderen Farbe grafisch zu codieren (306, 308).

7. System nach einem der Ansprüche 1 bis einschließlich 6, wobei die anatomische Karte (302) eine elektroanatomische Karte (EA-Karte) ist.

## Revendications

1. Système (10), comprenant :
un dispositif d'affichage (27) ; et
un processeur (56) qui est configuré pour :
recevoir une carte anatomique (302) de tissu de paroi d'au moins une partie d'une cavité cardiaque ; et
**caractérisé en ce que** le processeur est en outre configuré pour :
recevoir des emplacements d'électrodes (26) d'un cathéter (14) à l'intérieur de la cavité cardiaque pendant l'application de l'ablation ;
projeter (240) au moins certains des emplacements des électrodes sur une surface (202) de la carte ;
trouver des chemins les plus courts entre des paires d'emplacements projetés (230) d'électrodes (26) adjacentes ;
indiquer certains des chemins avec des premières indications (250, 252) d'ablation continue, lorsque les chemins (260) ont des longueurs de chemin qui sont inférieures à une longueur de chemin seuil donnée (255) ;
indiquer d'autres des chemins avec des secondes indications (254) d'espaces d'ablation sur la base de la géométrie de cathéter ;
générer des marqueurs d'ablation en grille (304) selon les première et seconde indications et un modèle d'ablation ;
superposer les marqueurs d'ablation en grille (304) sur la carte anatomique (302) pour générer une carte d'ablation (300) ;
coder de manière graphique (306, 308) la carte d'ablation selon les première et seconde indications des chemins ; et
présenter la carte d'ablation (300) codée de manière graphique (306, 308) à un utilisateur sur le dispositif d'affichage (27).

2. Système selon la revendication 1, dans lequel le processeur est configuré pour projeter (240) les emplacements en vérifiant une distance (241) entre l'emplacement et l'emplacement projeté (230) respectif, et en rejetant un ou plusieurs des emplacements projetés (240) dont la distance (241) dépasse une distance de projection seuil (242).

3. Système selon l'une quelconque des revendications 1 et 2, dans lequel le processeur est configuré pour trouver les chemins (260) les plus courts en trouvant des courbes géodésiques sur la surface (202) de la carte anatomique.

4. Système selon l'une quelconque des revendications 1 à 3, dans lequel le processeur est configuré pour indiquer (254) les chemins avec les secondes indications d'espaces d'ablation sur la base de la géométrie de cathéter (14) en indiquant, pour un cathéter à boucle (28), un chemin généré par une projection d'une électrode la plus proximale (26) du cathéter et d'une électrode la plus distale (26) du cathéter en tant qu'espace d'ablation.

5. Système selon l'une quelconque des revendications 1 à 4, dans lequel le processeur est configuré pour générer les marqueurs d'ablation en grille (304) en utilisant un modèle dipôle.

6. Système selon l'une quelconque des revendications 1 à 5, dans lequel le processeur est configuré pour coder de manière graphique (306, 308) la carte d'ablation (300) en colorant des marqueurs d'ablation (304) sur une région continue avec une couleur et en colorant des marqueurs d'ablation sur un espace d'ablation avec une autre couleur.

7. Système selon l'une quelconque des revendications 1 à 6, dans lequel la carte anatomique (302) est une carte électroanatomique (EA).
